# EUROPEAN PATENT APPLICATION

(11) **EP 4 523 700 A1**
(43) Date of publication of application: **19.03.2025**
(21) Application number: 23382934.0
(22) Date of filing: 14.09.2023
(51) Int. Cl.: A61K 38/44, A61K 36/185, A61K 36/42, A61K 36/889, A61K 36/899, A61P 13/02, A61P 13/10

(54) **DIAMINE OXIDASE (DAO) FOR USE IN THE TREATMENT AND/OR PREVENTION OF LOWER URINARY TRACT SYMPTOMS (LUTS), OVERACTIVE BLADDER (OAB) SYMPTOMS AND ENURESIS**

(71) Applicant: Dr Healthcare España, S. L., 08017 Barcelona (ES)
(72) Inventor: Duelo Riu, Juan José, 08017 Barcelona (ES); Ponce Díaz-Reixa, José Luis, 15006 A Coruña (ES); de Lecea Flores de Lemus, Carlos, 08023 Barcelona (ES); Tintoré Gazulla, Maria, 08021 Barcelona (ES); Cuñé Castellana, Jordi, 08191 Rubí (ES)
(74) Representative: Ponti & Partners, S.L.P

(57) **Abstract**

The present invention relates to diamine oxidase (DAO) for use in the treatment and/or prevention of lower urinary tract symptoms (LUTS), overactive bladder symptoms (OAB) and enuresis.

## Description

### Field of the invention

The present invention relates to diamine oxidase (DAO) for use in the treatment and/or prevention of lower urinary tract symptoms (LUTS), overactive bladder (OAB) symptoms and enuresis.

### Background

Lower urinary tract symptoms (LUTS) and overactive bladder (OAB) are a common complaint in adults (men and women) with a major impact on quality of life (QoL) and have a substantial economic burden (see J.N. Cornu et al. EAU Guidelines on Non-Neurogenic Male Lower Urinary Tract Symptoms (LUTS), incl. Benign Prostatic Obstruction (BPO), update march 2023, or https://uroweb.org/guidelines/management-of-non-neurogenic-male-luts, and www.nature.com/articles/s41598-020-76846-0,www.mayoclinic.org/diseases-conditions/overactive-bladder/symptoms-causes/syc-20355715).

Among the most common symptoms of LUTS, the following can be mentioned:
- Poor or intermittent urine stream
- Straining to pee
- Sudden urge to urinate
- Waking up many times at night to pee
- Having to pee frequently
- Feeling like you can't fully empty your bladder

Patients suffering from OAB:
- Feel a sudden urge to urinate that's difficult to control
- Experience unintentional loss of urine immediately after an urgent need to urinate (urgency incontinence)
- Urinate frequently, usually eight or more times in 24 hours
- Wake up more than two times in the night to urinate (nocturia)
- Unintentional urinary loss at night (enuresis in children)

Overactive bladder happens when the muscles of the bladder start to contract on their own even when the volume of urine in your bladder is low. These are called involuntary contractions, and they create an urgent need to urinate.

As shown in Section 3 of such document, lower urinary tract symptoms can be divided into storage, voiding, and post-micturition symptoms, and they are prevalent, cause bother and impair QoL. An increasing awareness of LUTS and OAB and storage symptoms in particular, is warranted to discuss management options that could increase QoL. Lower urinary tract symptoms affect both sexes and all age groups; consequently, their associated costs and burden are likely to increase with future demographic changes. Lower urinary tract symptoms are also associated with a number of modifiable risk factors, suggesting potential targets for prevention (e.g. metabolic syndrome). In addition, men with moderate-to-severe LUTS may have an increased risk of major adverse cardiac events.

More than half of the aged population, men and women, have at least one symptom of the low urinary tract which corresponds to LUTS or OAB; however, symptoms are often mild or not very bothersome. The prevalence of LUTS and OAB increases with age in both men and women. Lower urinary tract symptoms can progress dynamically: for some individuals symptoms persist and progress over long time periods, and for others they remit. Symptoms sometimes vary in intensity over periods of time. Lower urinary tract symptoms have traditionally been related to bladder outlet obstruction (BOO), most frequently when histological Benign Prostatic Hyperplasia (BPH) progresses through benign prostatic enlargement (BPE) to Benign Prostatic Obstruction (BPO). However, increasing numbers of studies have shown that LUTS are often unrelated to the prostate. Bladder dysfunction may also cause LUTS, including detrusor overactivity/overactive bladder, detrusor underactivity (DU)/underactive bladder (UAB), as well as other structural or functional abnormalities of the urinary tract and its surrounding tissues. Prostatic inflammation also appears to play a role in BPH pathogenesis and progression. In addition, many non-urological conditions also contribute to urinary symptoms, especially nocturia.

The symptoms of OAB can be sometimes gathered and are then representative of several conditions which are considered as conditions related to OAB. A non-exhaustive list of these conditions is as follows:
- Painful bladder syndrome;
- Nocturia and enuresis;
- Interstitial cystitis;

Other signs and symptoms associated with OAB or LUTs are as follows:
- Acute retention of urine is defined as a painful, palpable or percussible bladder, when the patient is unable to pass any urine.
- Chronic retention of urine is defined as a non-painful bladder, which remains palpable or percussible after the patient has passed urine. Patients with such symptoms may experience incontinence and may also have dysfunction in their upper urinary tract.
- Bladder outlet obstruction is the generic term for obstruction during voiding and is characterised by increasing detrusor pressure and reduced urine flow rate. It is usually diagnosed by studying the synchronous values of flow-rate and detrusor pressure.
- Benign prostatic obstruction is a form of BOO and may be diagnosed when the cause of outlet obstruction is known to be BPE. In the Guidelines the term BPO or BOO is used as reported by the original studies.
- Benign prostatic hyperplasia is a term used (and reserved) for the typical histological pattern, which defines the disease.
- Detrusor overactivity is a urodynamic observation characterised by involuntary detrusor contractions during the filling phase which may be spontaneous or provoked. Detrusor overactivity is usually associated with OAB syndrome characterised by urinary urgency, with or without urge urinary incontinence (UUI), usually with increased daytime frequency and nocturia, if there is no proven infection or other obvious pathology.
- Detrusor underactivity during voiding is characterised by decreased detrusor voiding pressure leading to a reduced urine flow rate. Detrusor underactivity causes OAB syndrome which is characterised by voiding symptoms similar to those caused by BPO.

Several conditions may contribute to signs and symptoms of overactive bladder, including:
- Neurological disorders, such as stroke and multiple sclerosis
- Diabetes
- Urinary tract infections that can cause symptoms similar to those of an overactive bladder
- Hormonal changes during menopause in women
- Conditions affecting the bladder, such as tumors or bladder stones
- Factors that get in the way of urine leaving the bladder, such as enlarged prostate, constipation or previous surgery to treat incontinence

Overactive bladder symptoms may also be associated with:
- Medications that cause your body to make a lot of urine or require that you take them with lots of fluids
- Drinking too much caffeine or alcohol
- Declining cognitive function due to aging, which may make it more difficult for your bladder to understand the signals it receives from your brain
- Difficulty walking, which can lead to bladder urgency if you're unable to get to the bathroom quickly
- Incomplete bladder emptying, which may lead to symptoms of overactive bladder, as you have little urine storage space left

While symptoms of overactive bladder (OAB) and related conditions are primarily prevalent in the adult population, children can also experience symptoms of OAB and nocturnal enuresis.

In order to proceed with a proper diagnostic evaluation of LUTS and OAB, the following tools are currently employed:
- Medical history
- Symptom score questionnaires
- Frequency volume charts and/or bladders diaries
- Physical examination and digital-rectal examination
- Urinalysis
- Measurement of prostate-specific antigen (PSA)
- Renal function measurement
- Post-void residual urine
- Uroflowmetry
- Imaging
- Urethrocystoscopy
- Urodynamics
- Non-invasive tests in diagnosing bladder outlet obstruction in men with LUTS

Up to date some of the current treatments for LUTS and OAB are as follows (See also Section 5 of previous reference):
- Conservative treatment based on a watchful waiting and modifications in behaviour and diet.
- Pharmacological treatment with alpha 1-Adrenoceptor antagonists (α1-blockers)
- Pharmacological treatment with 5α-reductase inhibitors
- Pharmacological treatment with muscarinic receptor antagonists
- Pharmacological treatment with beta-3 agonists
- Pharmacological treatment with phosphodiesterase 5 inhibitors
- Pharmacological treatment with inhibitors or mastocyte degranulation
- Phytotherapy with for example phytosterols, β-sitosterol, fatty acids and lectins.
- Combination therapies using one or more of the previous treatments
- Surgical treatment
- Resection of the prostate
- Enucleation of the prostate
- Vaporisation of the prostate
- Ablative techniques

Obviously, the first choice of therapy is always a conservative treatment but when this is not successful, other alternatives are then considered. In order to choose the best option for the treatment, the physician should consider aspects such as: previous patient evaluation, ability of the treatment to change the findings, treatment preferences of the individual patient and the expectations in terms of speed of onset, efficacy, side effects, QoL and disease progression.

The main problems with pharmacotherapy in LUTS and OAB are associated with its moderate efficacy, low persistence on treatment, and the incidence of short and long-term adverse events associated with some compounds. The number of compounds used in the pharmacological treatment of LUTS of patients who dot respond to conservative measures has been relatively stable and different combinations have been investigated to improve efficacy of standalone treatments or reduce their dose and potential side effects.

The long-term adverse events, such as cognitive impairment in the elderly vulnerable patients associated with antimuscarinic drugs or persistent erectile dysfunction in sexually active men after treatment with 5-alfa-reductase inhibitors, are some of the frequent problems seen in the clinic. Moreover, the interactions of these drugs with other medications which the elder population affected may be taken for cardiovascular, respiratory or neurological conditions require the use of new options which may be more physiological.

The function of the LUT is dependent upon the activity of the smooth muscle in the bladder and striated muscles present in the urethral sphincter and pelvic floor. These structures are a functional unit controlled by an interplay between the central and peripheral nervous systems and local regulatory factors. Diamine oxidase (DAO), a naturally occurring enzyme plays a role in facilitating a physiological control of this functional unit. Its natural origin and outstanding safety profile may help in improving the adherence of patients to long-term treatments.

Accordingly, the problem to be solved by the present invention is to find an alternative way for treating, improving and preventing lower urinary tract symptoms. The present invention solves this problem by employing DAO.

### Summary of the invention

In a first aspect, the present invention relates to diamine oxidase (DAO) for use in the treatment and/or prevention of LUTS, OAB and enuresis.

### Brief description of the drawings

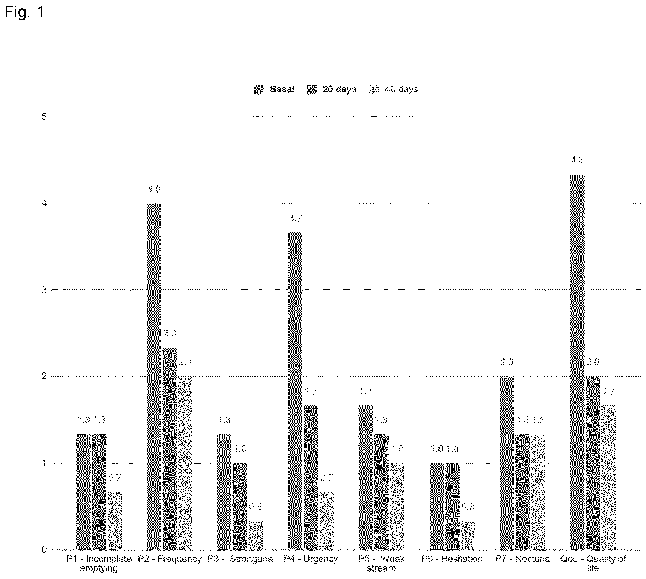

Fig. 1 shows how (in average) data of the patients diagnosed of LUTS by urology specialist according to independent parameters of the IPSS (International Prostate Symptoms Score). Basal data correspond to left bars, data after 20 days of supplementation with DAO correspond to central bars and data after 40 days of supplementation with DAO are shown on right bars.

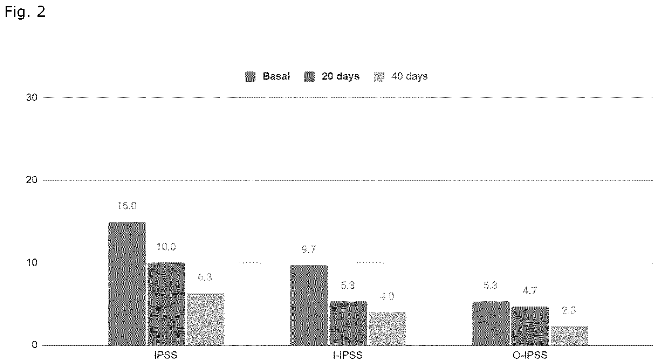

Fig. 2 shows the overall IPSS score and the score related to irritability (I-IPSS) and obstruction (O-IPSS). Basal data correspond to left bars, data after 20 days of supplementation with DAO correspond to central bars and data after 40 days of supplementation with DAO are shown on right bars.

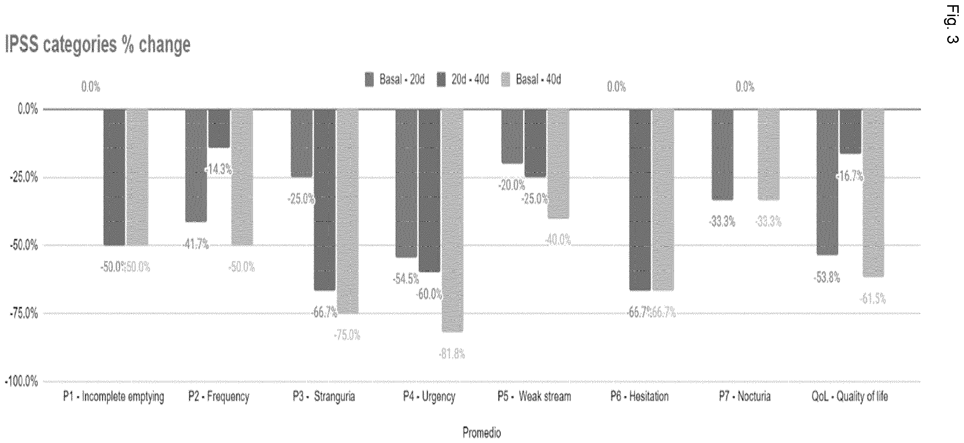

Fig. 3 show the percentage changes in symptom modification between the baseline situation and the evolution after 20 days of supplementation with DAO with respect to fig. 1 (left bars), between the day 20 and the day 40 of supplementation with DAO (central bars) and between the baseline situation and the evolution after 40 days of supplementation with DAO (right bars).

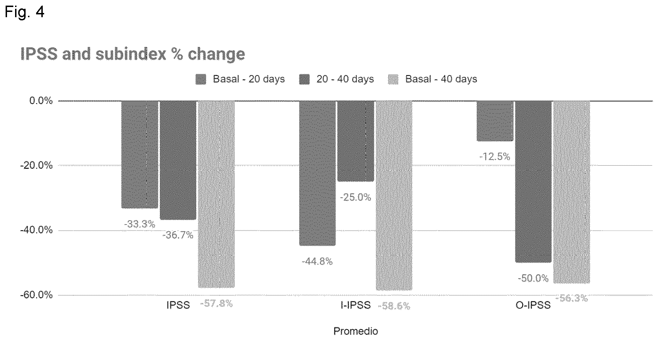

Fig. 4 show the percentage changes in symptom modification between the baseline situation and the evolution after 20 days of supplementation with DAO with respect to fig. 2 (left bars), between the day 20 and the day 40 of supplementation with DAO (central bars) and between the baseline situation and the evolution after 40 days of supplementation with DAO (right bars).

### Detailed description

The present invention relates to diamine oxidase (DAO) for use in the treatment and/or prevention of lower urinary tract symptoms (LUTS), OAB and enuresis. Preferably, the symptom among the lower urinary tract symptoms to be treated and/or prevented is voiding symptoms.

"DAO" is the abbreviation used to designate the enzyme diamine oxidase responsible for the catalysis of the oxidative deamination of the primary amine group of histamine to give imidazole acetaldehyde. DAO in the present invention may have different origin, e.g. a non-plant origin, plant origin or biotechnological origin. "Non-plant origin" means any DAO that is not obtained from plants but from animal organisms or other non-plant organisms. Thus, this definition includes all DAO isolated from living creatures that are not plants. "Plant origin" means any DAO obtained from plant organisms. "Biotechnological origin" means any DAO prepared from recombinant cell cultures or in non-plant organisms of any type after isolating the DNA for DAO.

"LUTS" as defined above makes reference to lower urinary tract symptoms, whose most common symptoms are:
- Poor or intermittent urine stream
- Straining to pee
- Sudden urge to urinate
- Waking up many times at night to pee
- Having to pee frequently
- Feeling like you can't fully empty your bladder
- Unintentional urinary loss in bed

As also mentioned above, lower urinary tract symptoms can be divided into storage, voiding, and post-micturition symptoms. Preferably, the present invention relates to storage and voiding symptoms.

"OAB" as defined above refers to a syndrome that is characterised by symptoms of urgency, with or without urge urinary incontinence, usually with frequency and nocturia/enuresis. It does include bladder pain syndromes such as interstitial cystitis.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

It should be noted that the term "approximately" or "about" applied to the values used earlier and later in this document includes a margin of error of ± 5 %, such as, for example, ± 4 %, ± 3 %, ± 2 %, ± 1 %.

As used herein, the term "treat" or "treatment" or "treating" and its cognates refer to an amelioration/improvement of at least one discernible symptom of LUTS and/or OAB and/or enuresis. In another embodiment, "treat" refers to an amelioration/improvement of at least one measurable physical parameter, not necessarily discernible by the patient, in another embodiment, "treat" or "treatment" or "treating" refer to inhibiting the progression of at least one discernible symptom of LUTS and/or OAB and/or enuresis, either physically (e.g., stabilization of a discernible symptom), physiologically (e.g., stabilization of a physical parameter), or both. In another embodiment, "treat" or "treatment" or "treating" refer to slowing the progression or reversing the progression of at least one discernible symptom of LUTS, OAB or enuresis. As used herein, "prevent" or "prevention" and its cognates refer to delaying the onset or reducing the risk of acquiring at least one discernible symptom of LUTS, OAB or enuresis.

As used herein, the term "preventing and/or treating" includes "preventing and treating" and "preventing or treating".

As used herein the term "supplementation" includes "dietary management", "administration of food supplements, dietary supplements or food for special medical purposes".

In another embodiment, said diamine oxidase (DAO) is combined with at least one another active ingredient. Said at least another active ingredient might be a compound selected from the group consisting of phosphodiesterase 5 inhibitors, 5 alfa-reductase inhibitors, alfa1 adrenoreceptor antagonists, beta3 receptor agonists, antimuscarinic or anticholinergic drugs, inhibitors of mastocyte degranulation, plant extracts and a combination thereof. Said plant extract is preferably selected from *Cucurbita pepo* (pumpkin seeds), *Hypoxis rooperi* (South African star grass), *Pygeum africanum* (bark of the African plum tree), *Secale cereale* (rye pollen), *Serenoa repens* or *Sabal serrulata* (saw palmetto), *Urtica dioica* (roots of stinging nettle), *Arctium lappa* (Burdock), *Capsella bursa-pastoris, Hedera helix, Hypericum perforatum, Ilex paraguariensis* (yerba mate, maté, kali chaye), *Lycopodium clavatum, Medicago sativa, Ononis spinosa, Pimpinella saxifrage, Echinacea purpurea* (Purple coneflower), *Echinacea angustifolia* (Echinacea, narrow-leaved purple coneflower root), *Justicia gendarussa, Cynara scolymus* (Artichoke leaf), *Prunus avium, Prunus cerasus* (Cherry Peduncle), *Calluna vulgaris* (Heather blossoming top), *Zea mays L.* (Corn silk), *Fraxinus excelsior* (Ash tree leaf), *Hieracium pilosella* (Mouse-ear Hawkweed, entire plant), *Camellia sinensis* (Tea leaf), *Betula pendula* / *Betula pubescens* / *Betula alba* (Birch), *Erica cinerea, Filipendula ulmaria* or *Spiraea ulmaria* (Meadowsweet), *Solidago virgaurea* (Golden herb), *Taraxacum officinale* (Dandelion), *Agathosma betulina* & *crenulata* (Buchu), *Epilobium parviflorum*/*angustifolium* or *Chamaenerion angustifolium* (Willow herb), *Ortosiphon stamineus, anistatus, spicatus* (Java tea), *Prunus africana* or *Pygeum africanum*), *Arctostaphylos uva ursi* (Bearberry), *Equisetum arvense* (Horsetail), *Asparagus racemosus* ROOT, *Asphaltum*/*Shilajit, Boerhaavia diffusa* ROOT, *Santalum album* HEARTWOOD, *Sida cordifolia* ROOT & WHOLE PLANT, *Solanum nigrum, Tribulus terrestris* FRUIT, *Agropyrum repens or Elytrigia repens, Anogeissus latifolia* (gum Combretaceae-Dhava-Axle Wood), *Lycopodium clavatum, Phyllanthus niruri*-herb-*Euphorbiaceae-Bhumyamalaki-*Laurel Seaside, *Polygonum aviculare herba* (Knotgrass), *Thymus serpyllum* (Herba Garden Thyme), *Cerasus avium stipites* (cherry stalk), *Phaseolus vulgaris fructus sine semine* (bean pod), *Achillea millefolium herba* (Yarrow herb), *Agropyron repens radix* (couch grass root), *Galium verum herba,* (lady's bedstraw herb), *Lavandula officinalis flos* (lavender flowers), *Mentha piperita herba* (peppermint herb), *Sambucus nigra flos, Foeniculum vulgare aetheroleum* (fennel essential oil), *Eucalyptus globulus aetheroleum* (eucalypt essential oil), *Abies alba aetheroleum* (silver fir essential oil), and *Matricaria recutita flos* (chamomile flower). More preferably, said plant extract is *Cucurbita pepo* (pumpkin seeds), *Hypoxis rooperi* (South African star grass), *Pygeum africanum* (bark of the African plum tree), *Secale cereale* (rye pollen), *Serenoa repens* (*saw palmetto*), *Urtica dioica* (roots of stinging nettle) or a combination thereof.

In a preferred embodiment, said diamine oxidase is included in a pharmaceutical composition. In a further preferred embodiment, said pharmaceutical composition further comprises a pharmaceutically acceptable vehicle, adjuvant, diluent or excipient, and optionally at least another active ingredient. Said pharmaceutically acceptable vehicle, adjuvant, diluent or excipient can be any of preserving agents, fillers, disintegrating agents, wetting agents, emulsifying agents, suspending agents, solvents, dispersion media, coatings, isotonic and absorption delaying agents and the like. The use of such media and agents for pharmaceutical active substances is well known in the art. Except insofar as any conventional media or agent is incompatible with the active ingredient, its use in the therapeutic compositions is contemplated.

In another preferred embodiment, said prevention and/or treatment comprises topically, orally, intravenously, intraperitoneally or by bladder instillation, preferably orally, administering said diamine oxidase or the pharmaceutical composition comprising thereof.

In case of bladder instillation, a compound selected from hyaluronic acid or other glycosaminoglycans, chondroitin sulphate, pentosan polysulphate sodium, oxychlorosense sodium, botulinum toxin A, capsaicin, resiniferatoxin, Bacillus Calmette Guerin (BCG), inhibitors of mastocyte degranulation or combinations thereof can be included with diamine oxidase or the pharmaceutical composition comprising thereof.

In a further preferred embodiment, the DAO or the pharmaceutical composition comprising thereof is administered orally. The dosage form may be selected from a tablet, microcapsule, nanocapsule, sachet, liposome and drink. In another preferred embodiment, said dosage forms may have gastric protection for protecting the DAO from gastric acidity. The enteric coating layer that coats the various forms rapidly disintegrates or dissolves in a neutral or alkaline medium. The enteric coating layers may contain pharmaceutically acceptable plasticisers to obtain the desired mechanical properties of flexibility and hardness. These plasticisers can be, for example, triacetin, citric acid esters, phthalic acid esters, cetyl alcohol, polyethylene glycols, polysorbates or other plasticisers.

The presence of the gastric protection would depend on the dosage form. For example, for topical administration or through bladder instillation this gastric protection is not necessary. Accordingly, the present invention encompasses dosage forms with and without gastric protection.

The above mentioned dosage forms may also contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavouring agent such as peppermint, or methyl salicylate. In addition, dosage unit forms can contain various other materials that modify the physical form of the dosage unit, for example, coatings of sugar, shellac, or enteric agents. Other oral dosage forms like syrup or elixir may contain sweetening agents, preservatives, dyes, colourings, and flavourings. In addition, the active compounds may be incorporated into fast dissolve, modified-release or sustained-release preparations and formulations, and wherein such sustained-release formulations are preferably bimodal.

DAO or the compositions according to the present invention are capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition.

DAO can also be preferably mixed with pharmaceutically acceptable alkaline substances such as salts of phosphoric acid and sodium, potassium, calcium, magnesium and aluminium, carbonic acid, citric acid or other suitably weak organic and inorganic acids; a co-precipitate of aluminium hydroxide/sodium bicarbonate; substances normally used in anti-acid preparations such as hydroxides of aluminium, calcium and magnesium; magnesium oxide or compound substances such as Al₂O₃.6MgO.CO₂.12H₂O, (Mg₆Al₂(OH)₁₆CO₃.4H₂O, MgO.Al₂O₃.2SiO₂.nH₂O or similar compounds; pH buffering substances such as tris(hydroxymethyl)aminomethane, basic amino acids and their salts or other pharmaceutically acceptable pH buffering substances.

In another preferred embodiment, said diamine oxidase is included in a functional food or a dietary supplement, alone or optionally combined with at least one another active ingredient as mentioned above. In other words, said diamine oxidase is included in food matrices providing them with the enzymatic activity (functional food) and helping in reducing their content in biogenic amines. Diamine oxidase could be incorporated in such food matrices during the process of elaboration (processing aid) or as an ingredient declared in the composition of the finished product. Said diamine oxidase included in a functional food or a dietary supplement can be present with any media or agent/compound compatible with the diamine oxidase and the optional at least one another active ingredient. Examples of such media or agent/compound are preserving agents, fillers, disintegrating agents, wetting agents, emulsifying agents, suspending agents, solvents, dispersion media, coatings, isotonic and absorption delaying agents and the like. Said functional food or dietary supplement is preferably orally administered.

"Alone" is meant to be as the unique active ingredient but including any acceptable vehicle, adjuvant, diluent or excipient suitable for such functional food or dietary supplement.

In another preferred embodiment, said diamine oxidase or the pharmaceutical composition comprising thereof is a medical device or is included in powder, semi-solid, semi-liquid or liquid form in a delivery system which is a medical device which facilitates the administration of the enzyme into de urinary system (i.e. catheters, tubes, endoscopic tools, instillation tools, etc.). Said diamine oxidase is present alone or optionally combined with at least one another active ingredient as mentioned above. Said diamine oxidase as a medical device or included in the medical device can be present with any media or agent/compound compatible with the diamine oxidase and the optional at least one another active ingredient. Examples of such media or agent/compound are preserving agents, fillers, disintegrating agents, wetting agents, emulsifying agents, suspending agents, solvents, dispersion media, coatings, isotonic and absorption delaying agents and the like.

The present disclosure also relates to a method of preventing and/or treating of lower urinary tract symptoms (LUTS), according to the embodiments included herein, comprising the step of administering a therapeutically effective amount or dose of DAO according to the embodiments included herein. The terms "therapeutically effective dose" and "therapeutically effective amount" are used to refer to an amount of the subject compound that results in prevention, delay of onset of symptoms, or amelioration of symptoms in the context of the present invention. A therapeutically effective amount, as well as a therapeutically effective frequency of administration, can be determined by methods known in the art and discussed below.

It is noted that any of the embodiments disclosed herein can be taken alone or combined with any other embodiment disclosed herein unless the context specifies otherwise. In other words, for example, a preferred option of a defined feature can be combined with a more or less preferred option of another feature.

The invention will be now illustrated by way of examples, which do not intend to limit its scope.

### EXAMPLES

### Example 1

Material and method: The present experiment aims to identify the relationship between genetic AOC1 polymorphism also known as genetic DAO deficiency and lower urinary tract symptoms (LUTS). After local ethic committee approval, one hundred subjects with moderate to severe LUTS were enrolled in a prospective pilot cohort study to identify prevalence of genetic DAO deficiency, described as at least one SNP affected in the AOC1 gene.

Results: 46% of subjects were men. Median age (SD) was 56.9 (14.6) years old. A high prevalence of 88% (91.3% in men; 85.2% in women) was identified in the enrolled patients, known as at least one SNPs with heterozygous mutation. Medium IPSS (International Prostate Symptoms Score) was 17.2 (6.9), with moderate (58%) and severe (35%) symptomatology. Men had significatively more voiding symptoms than women (p= 0.003), which had more severe storage symptoms, but not significatively (p=0.059). Genetic results showed that 20%, 22%, 15% and 31% of patients had 1, 2, 3 and 4 altered SNPs, without differences among sexes. Allelic distribution showed HW equilibrium. Genotypes showed higher prevalence of minor alleles expression associated with diminished DAO activity (c.691G>T 61%; p.Thr16Met 66%; p.Ser332.Phe 57% and p.His664Asp 59%). No differences among sexes were identified.

Minor alleles were associated with urinary tract symptom severity in c.691G>T (IPSS 14.9 vs 18.7, p=0.006) and p.Thr16Met 15.3 vs 18.3, p=0.027). Analyzing the symptoms category (moderate vs severe), we have shown a protective role of severe symptoms of female sex (men 45.7% vs female 25.9; OR 0.42, p=0.029). Minor alleles expression (either heterozygous or homozygous) of c.691G>T is associated with severe symptoms (OR 2.48 (1.01 - 6.10, p=0.046), as well as expression of minor alleles in both SNPs at same time (c.691G>T and p.Thr16Met - OR 2.98 (1.21 - 7.3, p=0.015) o even when all SNPs were affected (31% of our cohort) (OR 2.81 (1.16 - 6.77, p=0.020)

Looking for LUTS typology, we have identified significant differences among voiding or storage symptoms with severe voiding symptoms been significatively associated with the presence of minor alleles on c.691G>T (OR 17.0 (1.03 - 314.74); p=0.006) and p.Thr16Met (OR 14.3 (0.82 - 250.44); p=0.014). When these both SNPs (OR 20.5 (1.86 - 359.8); p=0.002) or all four SNPs had minor alleles (OR 4.74 (1.27 - 17.65); p=0.032) symptoms were more severe. Finally, homozygosity was also a risk factor for voiding symptom severity (OR 5.08 (1.24 - 20.77); p= 0.035). Storage symptoms were not associated globally with any minor alleles on the SNPs analyzed, although women tend to have more severe storage symptoms than men, but without significance (38,9% vs 21.7%, OR 2.29 (0.94 - 5.57), p=0.065).

### Conclusions

We have found a high prevalence of genetic DAO deficiency among subjects with LUTS reaching 88% and an important relationship with severity of voiding symptoms. To our knowledge this is the first evidence of a relationship between genetic DAO deficiency and LUTS.

### Example 2

Three subjects who had entered the study and who had DAO genetic alterations were treated with DAO. The treatment consisted of administering a tablet containing 4.2 mg of extract of pig kidney with at least 7 % p/p of DAO.

Fig. 1 shows how (in average) data of the patients diagnosed of LUTs by urology specialist according to independent parameters of the IPSS, which has 7 independent questions expressing the different symptoms of the lower urinary tract, has been modified after taking DAO enzyme for 20 and 40 days, respectively. As shown, some aspects substantially improve the score and most importantly, the scores drop below 3 already after 20 days (which is the limit to interpret this symptom as clinically significant) and continue improving after 40 days. That is, the symptoms only appear less than half of the times that the subject goes to the toilet. The overall quality of life improves as reflected by the reduction of the symptoms scores below 3 points (there is no dissatisfaction in the voiding quality of life).

Fig. 2 shows the overall IPSS score and the score related to irritability and obstruction. All aspects improve and the overall score drops from 15 to 10 after 20 days, and to 6.3 after 40, reaching the threshold from severe to mild symptoms (less than 9).

Finally, the percentage changes in symptom modification between the baseline situation and the evolution after 20 and 40 days of treatment are shown in figs 3 and 4.

These findings are new, relevant and important, since urinary symptoms were not previously associated with DAO deficiency. This could give new opportunities for better understanding of LUTS, sets the basis for future investigations and opens the door for supplementation with DAO enzymes in these type of patients.

## Claims

1. Diamine oxidase (DAO) for use in the treatment and/or prevention of lower urinary tract symptoms (LUTS), overactive bladder symptoms (OAB) and enuresis.

2. The diamine oxidase for use according to claim 1, wherein said lower urinary tract symptoms are voiding symptoms.

3. The diamine oxidase for use according to claim 1 or 2, wherein said DAO is combined with at least one another active ingredient.

4. The diamine oxidase for use according to claim 3, wherein said at least one another active ingredient is a compound selected from the group consisting of phosphodiesterase 5 inhibitors, 5 alfa-reductase inhibitors, alfa1 adrenoreceptor antagonists, beta3 receptor agonists, antimuscarinic or anticholinergic drugs, plant extracts, inhibitors of mastocyte degranulation, and a combination thereof.

5. The diamine oxidase for use according to claim 4, wherein said plant extract is selected from *Cucurbita pepo* (pumpkin seeds), *Hypoxis rooperi* (South African star grass), *Pygeum africanum* (bark of the African plum tree), *Secale cereale* (rye pollen), *Serenoa repens* or *Sabal serrulata* (saw palmetto), *Urtica dioica* (roots of stinging nettle), *Arctium lappa* (Burdock), *Capsella bursa-pastoris, Hedera helix, Hypericum perforatum, Ilex paraguariensis* (yerba mate, maté, kali chaye), *Lycopodium clavatum, Medicago sativa, Ononis spinosa, Pimpinella saxifrage, Echinacea purpurea* (Purple coneflower), *Echinacea angustifolia* (Echinacea, narrow-leaved purple coneflower root), *Justicia gendarussa, Cynara scolymus* (Artichoke leaf), *Prunus avium, Prunus cerasus* (Cherry Peduncle), *Calluna vulgaris* (Heather blossoming top), *Zea mays L.* (Corn silk), *Fraxinus excelsior* (Ash tree leaf), *Hieracium pilosella* (Mouse-ear Hawkweed, entire plant), *Camellia sinensis* (Tea leaf), *Betula pendula* / *Betula pubescens* / *Betula alba* (Birch), *Erica cinerea, Filipendula ulmaria* or *Spiraea ulmaria* (Meadowsweet), *Solidago virgaurea* (Golden herb), *Taraxacum officinale* (Dandelion), *Agathosma betulina & crenulata* (Buchu), *Epilobium parviflorum*/*angustifolium* or *Chamaenerion angustifolium* (Willow herb), *Ortosiphon stamineus, anistatus, spicatus* (Java tea), *Prunus africana* or *Pygeum africanum), Arctostaphylos uva ursi* (Bearberry), *Equisetum arvense* (Horsetail), *Asparagus racemosus* ROOT, *Asphaltum*/*Shilajit, Boerhaavia diffusa* ROOT, *Santalum album* HEARTWOOD, *Sida cordifolia* ROOT & WHOLE PLANT, *Solanum nigrum, Tribulus terrestris* FRUIT, *Agropyrum repens or Elytrigia repens, Anogeissus latifolia* (gum Combretaceae-Dhava-Axle Wood), *Lycopodium clavatum, Phyllanthus niruri-herb-Euphorbiaceae-Bhumyamalaki-*Laurel Seaside, *Polygonum aviculare herba* (Knotgrass), *Thymus serpyllum* (Herba Garden Thyme), *Cerasus avium stipites* (cherry stalk), *Phaseolus vulgaris fructus sine semine* (bean pod), *Achillea millefolium herba* (Yarrow herb), *Agropyron repens radix* (couch grass root), *Galium verum herba,* (lady's bedstraw herb), *Lavandula officinalis flos* (lavender flowers), *Mentha piperita herba* (peppermint herb), *Sambucus nigra flos, Foeniculum vulgare aetheroleum* (fennel essential oil), *Eucalyptus globulus aetheroleum* (eucalypt essential oil), *Abies alba aetheroleum* (silver fir essential oil), and *Matricaria recutita flos* (chamomile flower).

6. The diamine oxidase for use according to claim 5, wherein said plant extract is *Cucurbita pepo* (pumpkin seeds), *Hypoxis rooperi* (South African star grass), *Pygeum africanum* (bark of the African plum tree), *Secale cereale* (rye pollen), *Serenoa repens* (*saw palmetto*), and *Urtica dioica* (roots of stinging nettle) or a combination thereof.

7. The diamine oxidase for use according to any of the preceding claims, wherein said diamine oxidase is included in a pharmaceutical composition.

8. The diamine oxidase for use according to claim 7, wherein said pharmaceutical composition further comprises a pharmaceutically acceptable vehicle, adjuvant, diluent or excipient, and optionally at least another active ingredient.

9. The diamine oxidase for use according to any of the preceding claims, wherein said prevention or treatment comprises topically, orally, intravenously, intraperitoneally administering or administration by bladder instillation of said diamine oxidase or the pharmaceutical composition comprising thereof.

10. The diamine oxidase for use according to claim 9, wherein said prevention or treatment comprises orally administering said diamine oxidase or the pharmaceutical composition comprising thereof.

11. The diamine oxidase for use according to claim 9, wherein said diamine oxidase or the pharmaceutical composition comprising thereof is administered orally in a dosage form selected from a tablet, microcapsule, nanocapsule, sachet, liposome and drink.

12. The diamine oxidase for use according to claim 11, wherein said dosage forms have gastric protection.

13. The diamine oxidase for use according to claim 9, wherein the DAO or the pharmaceutical composition comprising thereof is administered by bladder instillation along with a compound selected from hyaluronic acid or other glycosaminoglycans, chondroitin sulphate, pentosan polysulphate sodium, oxychlorosense sodium, botulinum toxin A, capsaicin, resiniferatoxin, Bacillus Calmette Guerin, inhibitors of mastocyte degranulation, or combinations thereof.

14. The diamine oxidase for use according to any of the claims 1-6, wherein said diamine oxidase is included in a functional food or a dietary supplement.

15. The diamine oxidase for use according to any of the claims 1-8, wherein said diamine oxidase is a medical device or is included in powder, semi-solid, semi-liquid or liquid form in a delivery system which is a medical device.
